# EUROPÄISCHE PATENTSCHRIFT

(11) **EP 2 254 698 B1**
(45) Veröffentlichungstag und Bekanntmachung des Hinweises auf die Patenterteilung: **13.05.2015**
(21) Anmeldenummer: 09709694.5
(22) Anmeldetag: 16.02.2009
(51) Int. Cl.: B01L 3/00, B01L 7/00

(54) **MOBILES GERÄT FÜR DIE NUKLEINSÄUREISOLIERUNG**
MOBILE DEVICE FOR THE ISOLATION OF NUCLEIC ACID
APPAREIL MOBILE POUR ISOLEMENT D'ACIDES NUCLÉIQUES

(30) Priorität: 15.02.2008 DE 102008009920
(43) Veröffentlichungstag der Anmeldung: 01.12.2010
(73) Patentinhaber: Aj Innuscreen Gmbh, 13125 Berlin (DE)
(72) Erfinder: HILLEBRAND, Timo, 15366 Hoppegarten (DE); KNIPPSCHILD, Claus, 07745 Jena (DE); JASCHINSKY, Benjamin, 06114 Halle/Saale (DE)
(74) Vertreter: Wehlan, Helmut
(86) Internationale Anmeldenummer: PCT/EP2009/051820
(87) Internationale Veröffentlichungsnummer: WO 2009/101211

(56) Entgegenhaltungen:
- EP-A- 0 342 155
- WO-A-2007/099311
- DE-U1- 20 300 998
- US-A- 5 589 136
- US-A1- 2005 002 024
- US-A1- 2007 077 648

## Beschreibung

Gegenstand der Erfindung ist ein mobiles Gerätesystem, umfassend ein Handgerät und ein Testbesteck zur mobilen Isolierung von Nukleinsäuren.

### Stand der Technik

Die Untersuchung diagnostisch relevanter biologischer Proben wie Serum, Plasma, Blut, Tupferproben oder Organabriebe zum Nachweis infektiöser Erreger hat in den letzten Jahren enorm an Bedeutung gewonnen. Virusinfektionen wie HIV, HCV oder HBV sind weltweit auf dem Vormarsch. Darüber hinaus sind auch bakterielle Infektionen wieder auf dem Vormarsch, u.a. auch im Ergebnis beginnender klimatischer Veränderungen. Das Auftreten neuer, tödlich verlaufender Infektionskrankheiten mit einem extrem hohen Infektiösitätspotential (SARS, Vogelgrippe) zeiget immer deutlicher, dass eine schnell und vor Ort durchführbare Diagnostik entscheidend für die Verhinderung von Epidemien sein wird. Dies betrifft auch die Problematik von Bioterrorismus und einem schnellen Vor-Ort-Nachweis von Erregern, die als biologische Kampfstoffe eingesetzt werden können.

Diesen Problemen Rechnung zu tragen, gibt es eine Reihe von Entwicklungen, die sich mit der so genannten Vor-Ort-Diagnostik beschäftigen. Dabei soll es sich um Geräte handeln, die alle Schritte der molekularen Diagnostik (Nukleinsäureisolierung, Amplifikation und Detektion) vereinen. Diese Entwicklungen fokussieren auf den Bereich der militärischen Diagnostik und sind auch in Analogie zu den klassischen "REAL-TIME-PCR-Verfahren" sehr teuer, sowohl geräte- als auch reagenzienseitig.

Es erscheint realistisch, dass die Schritte der Amplifikation und Detektion lösbar sind. Die größten Probleme bereitet aber die Integration der Probenvorbereitung. Der Grund dafür ist sehr einfach und begründet sich aus der Problematik von zu untersuchenden unterschiedlichsten und vor allem "problematischen" Ausgangsmaterialien. Das Problem einer Vor-Ort-Nukleinsäureisolierung ist damit nicht gelöst.

Unter klassischen Bedingungen erfolgt die Isolierung von DNA aus Zellen und Geweben dadurch, dass die Nukleinsäuren enthaltenden Ausgangsmaterialien unter stark denaturierenden und reduzierenden Bedingungen, teilweise auch unter Verwendung von proteinabbauenden Enzymen aufgeschlossen, die austretenden Nukleinsäurefraktionen über Phenol-/Chloroform- Extraktionsschritte gereinigt und die Nukleinsäuren mittels Dialyse oder Ethanolpräzipitation aus der wässrigen Phase gewonnen werden (Sambrook, J., Fritsch, E.F. und Maniatis, T., 1989, CSH, "Molecular Cloning"). Diese "klassischen Verfahren" zur Isolierung von Nukleinsäuren aus Zellen und besonders aus Geweben sind sehr zeitaufwendig (teilweise länger als 48 h) und erfordern einen erheblichen apparativen Aufwand. Verschiedene alternative Verfahren zur Isolierung von Nukleinsäuren aus unterschiedlichen biologischen Ausgangsmaterialien ermöglichen die aufwendige und gesundheitsschädigende Phenol-/Chloroform- Extraktion von Nukleinsäuren zu umgehen sowie eine Reduzierung der zeitlichen Aufwendungen zu erreichen. Alle diese Verfahren basieren auf einer von Vogelstein und Gillespie (Proc. Natl. Acad. Sci. USA, 1979, 76, 615-619) entwickelten und erstmals beschriebenen Methode zur präparativen und analytischen Reinigung von DNA- Fragmenten aus Agarosegelen. Die Methode kombiniert die Auflösung der die zu isolierende DNA- Bande enthaltenden Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2]; 200mM NaCl; 2 mM EDTA; 50% v/v Ethanol) gewaschen und danach von den Trägerpartikeln abgelöst.

Diese Methode erfuhr bis heute eine Reihe von Modifikationen und wird zum gegenwärtigen Zeitpunkt für unterschiedliche Verfahren der Extraktion und Reinigung von Nukleinsäuren aus unterschiedlichen Herkünften angewendet (Marko, M.A., Chipperfield, R. und Birnboim, H.G., 1982, Anal. Biochem., 121, 382-387).

Darüber hinaus existieren heute weltweit auch eine Vielzahl von Reagenziensystemen, vor allem zur Reinigung von DNA- Fragmenten aus Agarosegelen und für die Isolierung von Plasmid DNA aus bakteriellen Lysaten, aber auch für die Isolierung von längerkettigen Nukleinsäuren (genomische DNA, zelluläre Gesamt- RNA) aus Blut, Geweben oder auch Zellkulturen. Alle diese kommerziell verfügbaren Kits basieren auf dem hinlänglich bekannten Prinzip der Bindung von Nukleinsäuren an mineralische Träger unter Anwesenheit von Lösungen unterschiedlicher chaotroper Salze und verwenden als Trägermaterialien Suspensionen feingemahlener Glaspulver (z.B. Glasmilk , BIO 101, La Jolla, CA), Diatomenerden (Fa. Sigma) oder auch Silicagele. (Diagen, DE 41 39 664 A1).

Ein für eine Vielzahl unterschiedlicher Anwendungen praktikables Verfahren zur Isolierung von Nukleinsäuren ist in US 5,234,809 (Boom) dargestellt. Dort ist ein Verfahren zur Isolierung von Nukleinsäuren aus nukleinsäurehaltigen Ausgangsmaterialien durch die Inkubation des Ausgangsmaterials mit einem chaotropen Puffer und einer DNA- bindenden festen Phase beschrieben. Die chaotropen Puffer realisieren sowohl die Lyse des Ausgangsmaterials als auch die Bindung der Nukleinsäuren an die feste Phase. Das Verfahren ist gut geeignet, um Nukleinsäuren aus kleinen Probenmengen zu isolieren und findet speziell im Bereich der Isolierung viraler Nukleinsäuren seine praktische Anwendung.

Spezifische Modifikationen dieser Verfahren betreffen den Einsatz von neuartigen Trägermaterialien, welche für bestimmte Fragestellungen applikative Vorteile zeigen (WO-A 95/34569). In neueren Patentschriften wird offenbart, dass für die Adsorption von Nukleinsäuren an die dem Fachmann bekannten und eingesetzten silikatischen Materialien auch sogenannte antichaotrope Salze als Bestandteil von Lyse/ Bindungspuffer- Systemen sehr effizient und erfolgreich eingesetzt werden können (EP 1135479). Vorteil dieses Verfahrens ist, dass durch die Umgehung der Verwendung von chaotropen Salzen eine deutlich geringere gesundheitliche Gefährdung von den Extraktionssystemen ausgeht. Allerdings werden für eine effiziente Isolierung von Nukleinsäuren aus einer komplexen biologischen Probe insbesondere in Hinblick auf eine möglichst ausbeutenstarke Nukleinsäure-Gewinnung im Lysepuffer wiederum hohe Salzkonzentrationen (> 1.5 M) benötigt. So offenbart die Patentschrift, dass die Verwendung findenden Lysepuffer Salzkonzentrationen zwischen 1,5 M - 3 M enthalten.

Diese wenigen Beispiele aus dem Stand der Technik machen deutlich, dass es eine Vielzahl von einfachen Verfahren zur Isolierung von Nukleinsäuren gibt. Insbesondere die Möglichkeit der Isolierung von Nukleinsäuren über deren Adsorption an geeignete Trägermaterialien (Glasfasermaterialen in Form von Filtern, Suspensionen aus silikatischen Komponenten oder unterschiedliche Arten von magnetischen oder paramagnetischen Partikeln) hat sich in der Laborroutine weltweit durchgesetzt. Dem Fachmann ist aber auch bekannt, dass die Durchführung von Nukleinsäureisolierungen, prinzipiell unabhängig von der Art der eingesetzten Chemie, immer an eine umfangreiche Laborausrüstung gebunden ist. Benötigt werden u.a. Zentrifugen, Vortexer, Thermomixer oder Inkubatoren, Separationsmodule für Magnetpartikel und ggf. auch Automaten. Um Nukleinsäuren auch unter Feldbedingungen und damit mit mobilen Systemen isolieren zu können, sind bisher nur Lösungswege beschritten worden, welche den Prozess der Isolierung von Nukleinsäuren mit einer nachfolgenden Amplifikation und Detektion verknüpfen. Ein solches Herangehen ist sehr kompliziert und in einer universellen Form auch bisher nicht gelöst. Darüber hinaus werden solche Gerätesysteme extrem teuer in der Anschaffung wie auch in Hinblick auf die benötigten Verbrauchsmaterialien. Einfache und vor allem preiswerte sowie universell nutzbare Verfahrenslösungen (bezogen auf unterschiedlichste biologische Proben) gibt es jedoch nicht.

Der Erfindung lag deshalb die Aufgabe zugrunde, die beschriebenen Nachteile der im Stand der Technik genannten Lösungen zu beseitigen.

Die Aufgabe wurde gemäß den Merkmalen der Patentansprüche gelöst.

Das erfindungsgemäße neuartige mobile System (Handgerät) ermöglicht auf einfache und preiswerte Weise die Isolierung von Nukleinsäuren aus unterschiedlichsten Ausgangsmaterialien, auch unter "Feldbedingungen" und ist darüber hinaus auch von "Nicht-Spezialisten" durchführbar.

Die Kombination von mobilem Kleingerät und Extraktionsreagenzien eröffnet erstmalig die Möglichkeit, die Diagnostik von Infektionskrankheiten in Entwicklungsländern ohne qualitative Einschränkungen durchführen zu können.

Die Erfindung löst die beschriebene Problematik in idealster Weise und ist hinsichtlich des Geräteaufbaus und der daraus resultierenden Einfachheit der Isolierung von Nukleinsäuren aus biologischen Proben auch für die Anwendung durch "Nicht-Spezialisten" unter Feldbedingungen geeignet. Darüber hinaus können sowohl die Gerätekosten als auch die benötigten Reagenzien sehr preiswert sein. Dies bedeutet einen enormen Vorteil gegenüber den konzipierten Gerätesystemen, insbesondere für militärische Anwendungen.

Die Erfindung löst das bestehende Problem in Bezug auf eine mobile und vor-Ort durchführbare Nukleinsäureisolierung überraschenderweise in ganz einfacher Form.

Die Erfindung nutzt dabei das hinlänglich bekannte Prinzip der Isolierung von Nukleinsäuren mittels magnetischer oder paramagnetischer Partikel. Der generelle Verfahrensablauf entspricht dem bereits geschilderten Stand der Technik. Er gliedert sich in die Prozesse:
1. Lyse des Ausgangsmaterials
2. Anbindung der freigesetzten Nukleinsäuren an magnetische oder paramagnetische Partikel
3. Waschen der oberflächengebundenen Nukleinsäuren
4. Trocknung der Partikel
5. Elution der gebundenen Nukleinsäure

Wie schon dargestellt, werden für die Durchführung dieser Verfahrensschritte unter Laborbedingungen z.B. ein Thermomixer oder Wasserbad (für die Lyse der Probe), ein Vortexer für das Durchmischen der Reaktionsansätze sowie eine Magnetseparator benötigt.

Die beschriebene Diversität der Ausgangsmaterialien (Probenstruktur und umgebende Matrix) verhinderte bisher die Integration eines Nukleinsäureaufreinigungsverfahrens mit einem Analyseverfahren.

Obwohl eine Vielzahl von Einmal-Probengefäßen zur Behandlung von einzelnen Matrizes bzw. Probenmaterialien existiert, gab es bisher kein mobiles System, das eine teilautomatisierte Prozessierung dieser verschiedenen Gefäße ermöglicht.

Solche Probengefäße erlauben z.B. die Aufnahme von Tupfern, sind mit Filtermembranen ausgestattet oder ermöglichen durch ihre innere Ausformung eine bessere Abtrennung von verschiedenen Probenphasen.

Die hier beschriebene Erfindung löst gestellte Aufgaben durch die Bereitstellung einer universellen Gerätebasis, einer Probenblockaufnahme und mit angepassten, funktionalisierten Probenblöcken (zum Einsetzen der Probengefäße bzw. Consumables) und angepasster, einfach handhabbarer Aufreinigungschemie. Dem Anwender wird damit die Möglichkeit eröffnet, durch manuelle Kombination dieser Bestandteile und verschiedener Consumables eine Matrix und probenspezifische Aufreinigung von Nukleinsäuren vorzunehmen.

Erstaunlicherweise ermöglicht gerade dieses Wechselblocksystem das große Spektrum an Probenmaterial und Anwendungen im Freiland zu bearbeiten.

Die Gerätebasis beinhaltet elektronische Steuereinheiten für die Blöcke, die Spannungsquelle sowie eine Verbindung zur Probenblockaufnahme.

Die Probenblockaufnahme beinhaltet eine Schüttel- bzw. Vibrationseinrichtung sowie eine oder mehrere reversiblen Verbindungsstellen für die Wechselblöcke. In einer weiteren Ausführungsvariante enthält dieses Element ggf. eine zusätzliche Heizeinrichtung.

Um ein leichtgängiges Ablösen der Blöcke von der Probenblockaufnahme bzw. der Probenblockaufnahme von der Gerätebasis zu gewährleisten, ist es denkbar, die Kontaktierung der beiden Elemente statt mit einem Steckverbinder mittels einer Magnetverbindung zu erreichen. Die Flexibilität des Gesamtgerätes kann durch das leichte Abnehmen des Blocks und der Probenblockaufnahme somit erheblich gesteigert werden.

Eine Ausführung der Erfindung schließt einen Probenblock mit Heizmodul ein, wobei die Geometrie des Blockes speziell auf die bereits beschriebenen Verbrauchsmaterialien angepasst ist. In einer weiteren Ausführungsvariante ist der beheizbare Probenblock mit einer Schüttelvorrichtung versehen.

Da der Großteil der Probenaufnahmebehälter (Consumables) aus einem durchsichtigen Polymer gefertigt sind, wird dem Anwender in einer weiteren Ausführungsvariante die visuelle Beurteilung des Aufreinigungsvorgangs -und damit die direkte, manuelle Einflussnahme auf den Prozess- durch ein Sichtfenster in dem Probenblock ermöglicht. Mittels der im Gerätesystem beigelegten Einmalpipetten erfolgt eine Zu- bzw. Abgabe von Flüssigkeiten. Somit ist eine kontrollierte, gesicherte Behandlung auch stark visköser Flüssigkeiten gewährleistet.

Ein weiterer Block dieses Wechselblocksystems kann ein Magnetseparationsblock sein. Dieser Magnetseparationsblock zeichnet sich dadurch aus, dass er während des Aufreinigungsprozesses von der Probenblockaufnahme abgenommen werden kann. Er verfügt über einen oder mehrere starke Magneten, die zur Separation der Magnetpartikel dienen und diese an der Wand des Consumables bewegen. Die anhaftenden Magnetpartikel müssen nun von der Flüssigkeit befreit werden. Durch die Abnehmbarkeit des Separationsblockes kann ein einfaches Abtrennen der Flüssigkeit erreicht werden. Dazu kann der Benutzer den Block einfach abnehmen, umdrehen. und die Flüssigkeit ablaufen lassen. Die starken Magneten halten die wichtigen Magnetpartikel fest, so dass kein Verlust entsteht.

In dieser Ausführungsform besitzt der Separationsblock ebenfalls Sichtfenster, so dass der Benutzer (wenn er das Gefäß nicht umdrehen möchte) einen Einblick in das Gefäß erhält und eine Abnahme der Flüssigkeit auch durch eine Einmalpipette ermöglicht wird. Der Benutzer ist somit in der Lage, den Separationsblock mit einem reversibel festgehaltenen Probengefäß in die Hand zu nehmen und ein sauberes Entfernen der Flüssigkeit vorzunehmen.

Erstaunlicherweise zeigte sich, dass bei verschiedenen Magnetpartikeln und verschiedenen Probenmatrizes die Anordnung der Magneten und die Stärke der Magneten eine sehr hohe Auswirkung auf die Separation hat.

Erstaunlicherweise zeigte sich bei der Verwendung des identischen Consumables und gleicher Anordnung eines Magnettyps eine erheblich abweichende Ausbeute von Nukleinsäuren auch bei nur gering abweichender Flüssigkeit z.B. verschiedener Viskositäten.

Hier kann durch eine optimale Anordnung der Magneten eine erhebliche Verbesserung der Ausbeuten erreicht werden, wobei die Stärke des oder der Magneten nicht unbedingt einen sehr großen Einfluss hatte.

Ebenfalls hat der Benutzer sehr einfach die Möglichkeit, ein Separationsmodel zu wechseln und in den Probenblock aufzustecken, somit das Feature eines ggf. geheizten und geschüttelten Separationsmoduls nicht verloren geht.

Durch Wechselbarkeit des Separationsmoduls und den gewonnenen Erkenntnissen bezüglich der Anordnung und Stärke der oder des Magneten sind verschiedenste Separationsmodule für unterschiedlichste Verfahren und Anwendungen denkbar. Ebenfalls ist eine Anpassung auf verschiedenste Consumables denkbar, bei der Aufrechterhaltung des mobilen Charakters des Gerätes.

Als Beispiel sei hier ein visköses Medium mit Magnetpartikeln aufgeführt, wobei eine Anordnung der Magneten so gewählt sein sollte, dass eine Durchdringung der Feldlinien angepasst auf das Consumable und den Flüssigkeitsstand eine schnelle und räumlich optimierte Trennung für dieses Verfahren und diese Matrix ermöglicht.

In dem die Probenblockaufnahme eine variable Schütteleinrichtung beinhaltet, kann die Funktionalität der einzelnen Wechselblöcke erhöht werden. So unterstützt das Schütteln z.B. die Separation der Magnetpartikel bei sehr viskösen Matrizes.

Eine Analyse der durch die Erfindung bereitgestellte, aufgereinigte Nukleinsäure kann dann durch geeignete Verfahren vor Ort oder im Labor erfolgen. Der Verbinder zwischen Gerätebasis und Probenblockaufnahme kann in einer weiteren Ausführungsvariante so gestaltet sein, dass nach dem Abnehmen der Probenblockaufnahme eine geeignete Analysevorrichtung aufgesteckt und mit der aufgereinigten Probe bestückt werden kann. Somit dient die Gerätebasis als mobile Grundversorgungseinrichtung für ein weiteres Werkzeugsystem.

Der schematische Aufbau des Handgerätes ist unter dem Ausführungsbeispiel schematisch dargestellt. Damit vereint das mobile Handgerät technisch all die benötigten technischen Spezifikationen, welche im Labor durch separate Einzelgeräte existieren (Thermomixer oder Inkubator, Vortexer, Magnetseparator). Mit dem erfindungsgemäßen Handgerät kann damit einfach und schnell die Isolierung von Nukleinsäuren aus unterschiedlichsten Proben vor Ort durchgeführt werden. Kombiniert wird das Handgerät noch mit einem Testbesteck, welches für eine Standard-Nukleinsäureisolierung benötigte Reagenzien sowie Einwegplastikmaterial enthält.

Der Ablauf der Nukleinsäureisolierung mittels des erfindungsgemäßen Handgerätes z.B. aus einem Rachenabstrich realisiert sich dann unter Feldbedingungen wie folgt:

### 1. Lyse der Probe:

Dazu Tupfer überführen in ein Reaktionsgefäß und Zugabe von Lysepuffer und ggf. einem proteolytschen Enzym mittels einer Einmalpipette bzw. mittels einer Tropfflasche. Optional können das Lysereagenz und das Enzym schon als lagerstabile Komponente am Reaktionsgefäß vorliegen, so dass dann nur noch Wasser zum Tupfer zugeführt werden muss. Überführen des Gefäßes in das Handgerät (in die Vertiefung des Heizblockes (oder des Heiz/ Schüttelblockes). Erwärmen des Heizblockes auf 30 - 95°C (in Abhängigkeit von der Anwendung) und ggf. schütteln des Gefäßes während der Lyse.

### 2. Anbindung der DNA an magnetische Partikel:

Dazu Zugabe eines Bindungspuffers mit Magnetpartikeln zum Lyseansatz und kurzes Schütteln und vollständiges Durchmischen von Bindungspuffer, Magnetpartikel und Lyseansatz.

### 3. Separation der Magnetpartikel mit der gebundenen Nukleinsäure:

Dazu Gefäß in die Vertiefung des Magnetseparationsmoduls stecken. Hier erfolgt die Sammlung der Magnetpartikel. Entfernen der restlichen Flüssigkeit mittels einer Einmalpipette oder durch Herausnehmen des Magnetblocks und einfachem Abgießen und Entsorgung in einer Abfallflasche.

### 4. Waschen der Magnetpartikel mit der gebundenen Nukleinsäure:

Dazu Zugabe eines Waschpuffers mittels einer Einmalpipette oder einer Tropfflasche zum Reaktionsgefäß mit den separierten Magnetpartikeln. Kurzes Schütteln des Reaktionsgefäßes und erneute Separation der Magnetpartikel durch Einsetzen des Reaktionsgefäßes in das Magnetseparationsmodul. Überstand wieder mittels einer Einwegpipette entfernen oder Herausnehmen des Magnetblocks und einfachem Abgießen und Entsorgung in einer Abfallflasche.

### 5. Trocknung der Magnetpartikel:

Dazu das Reaktionsgefäß wieder in den Heizblock umstecken und Inkubation für einige Minuten mit geöffnetem Deckel bei einer Temperatur von z.B. 30°C - 70°C.

### 6. Elution der Nukleinsäure von den Magnetpartikeln:

Dazu Zugabe eines Elutionspuffers oder Zugabe von Wasser mittels einer Einmalpipette oder einer Tropfflasche zum Reaktionsgefäß mit den getrockneten Magnetpartikeln. Kurzes Schütteln des Reaktionsgefäßes und erneute Separation der Magnetpartikel durch Einsetzen des Reaktionsgefäßes in das Magnetseparationsmodul. Der Überstand enthält die isolierte Nukleinsäure und kann in ein sauberes Reaktionsgefäß überführt werden.

Im Fall der alternativen Ausführung der Erfindung gemäß Anspruch 6 kann auf den Wechsel der des Reaktionsgefäßes zwischen dem Heiz- und Magnetseparationsmodul verzichtet werden. Die Sammlung der Magnetpartikel erfolgt in diesem Fall durch Einsetzen eines Magnetes in die Aussparung neben dem kombinierten Heiz-Magnetseparationsmodul oder durch Einschalten des Elektromagneten.

Die isolierte Nukleinsäure steht nun für vorgesehene Analyseverfahren sofort zur Verfügung.

Die einfache Handhabung des Handgerätes in der Kombination mit bereitgestellten Extraktionsreagenzien und einfach zu bedienendem Plastikzubehör ermöglicht damit die Durchführung einer Nukleinsäureisolierung unter Feldbedingungen. Die Einfachheit der notwendigen Verfahrensabläufe gestatten darüber hinaus die Durchführung der Nukleinsäureisolierung durch "Nichtspezialisten". Darüber hinaus kann die Isolierung von Nukleinsäuren prinzipiell aus jeder interessierenden Probe erfolgen. Die spezifische Anforderung wird durch spezifische Reagenzien- Kits realisiert. Das Handgerät bleibt dabei die konstante Komponente.

Auf Grund der technischen Einfachheit steht mit dem erfindungsgemäßen Handgerät für die mobile Nukleinsäureisolierung ein sehr preiswertes Gerätesystem zur Verfügung. Ein solches komplexes Handgerät kann damit auch in idealster Weise für diagnostische Tests in Entwicklungsländern eingesetzt werden. Die Universalität des Handgerätes ermöglicht darüber hinaus auch die Isolierung von Nukleinsäuren unter realen "Feldbedingungen" z.B. im Kontext mit militärmedizinischen Indikationen. Hierbei kann mit dem erfindungsgemäßen Handgerät eine Nukleinsäureisolierung universell und unabhängig von der Ausgangsprobe durchgeführt werden und die isolierte Nukleinsäure dann z.B. in portable Diagnosegeräte eingespeist werden.

Die nachfolgenden Beispiele skizzieren den möglichen Aufbau des Handgerätes zur mobilen Nukleinsäureisolierung und stellen keine Limitierung der Ausführung dar.

### Ausführungsbeispiel

### Beispiel 1

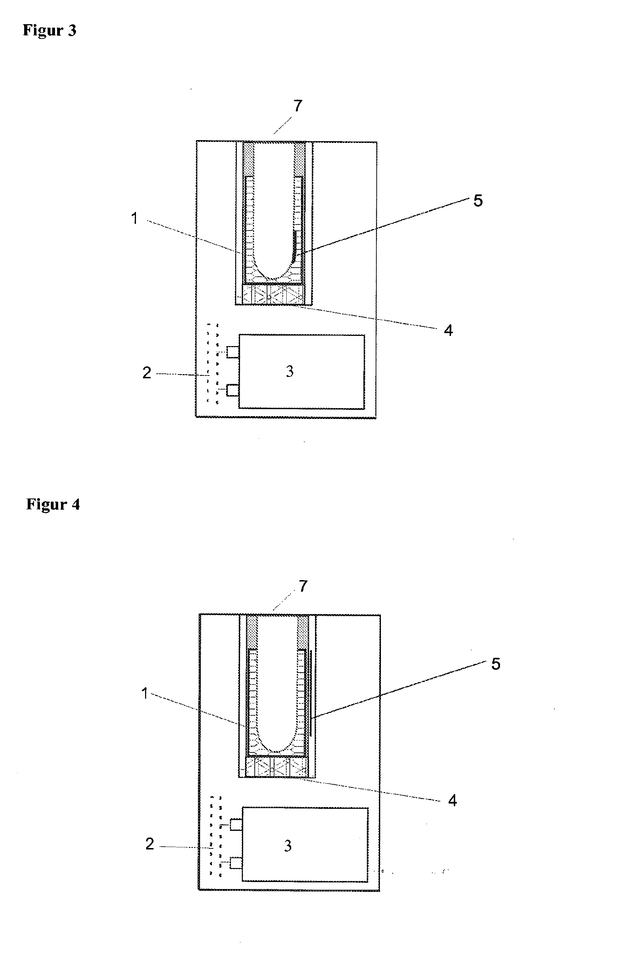

Eine Schematische Darstellung eines portablen Handgerätes zur Nukleinsäureisolierung für die mobile vor- Ort- Nukleinsäureisolierung und Testbesteck zur Durchführung des Extraktionsverfahrens zeigt Figur 2. Figuren 3 und 4 zeigen eine alternative Ausführungsform.

Das Testbesteck zur Vor-Ort-Nukleinsäureisolierung mittels des Handgerätes enthält:
1. Einwegpipetten (z.B. Pasteurpipetten)
2. Flasche für Reagenzienabfall
3. Suspension mit magnetischen oder paramagnetischen Partikeln
4. Reaktionsgefäße
5. Lysepuffer sowie optional proteolytische Enzyme in flüssiger Form oder Reaktionsgefäße für die Lyse der Probe mit lagerstabilen festen Formulierungen an Lysereagenzien und proteolytischen Enzymen
6. Bindungspuffer
7. Waschpuffer
8. Elutionspuffer

Vorzugsweise befinden sich alle Extraktionsreagenzien und die benötigten Einwegpipetten in einem Transportkoffer.

Die Durchführung einer spezifischen vor- Ort- Nukleinsäureisolierung erfolgt mittels des erfindungsgemäßen Handgerätes in der beschriebenen Ablauffolge.

### Beispiel: 2:

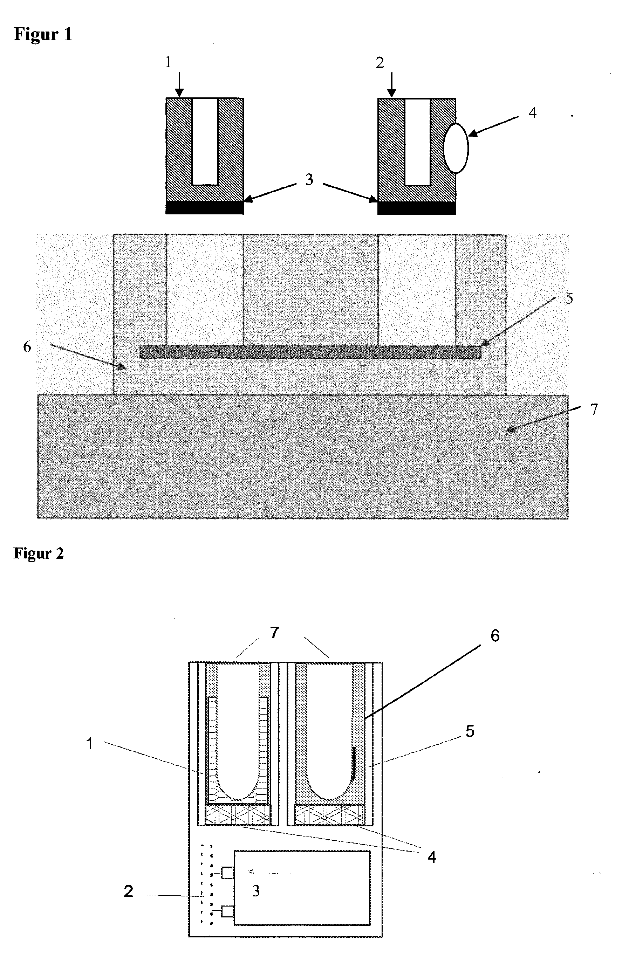

Figur 1 zeigt das erfindungsgemäße Gerät in einer bevorzugten Ausführungsform. Der Heizblock und der Magnetseparationsblock sind von der Probenblockaufnahme abnehmbar. Die Probenblockaufnahme enthält eine Schüttelvorrichtung und ggf. Heizelemente und ist vom Basisgerät abnehmbar.

### Bezugszeichenliste

**Figur 1****:**
   1 Heizblock
   2 Magnetseparationsblock
   3 Heizeinrichtung
   4 Magnet
   5 Schütteleinrichtung ggf. mit Heizelement
   6 Probenblockaufnahme
   7 Gerätebasis

| **Figur 2** | **Figur 3** | **Figur 4** |
|---|---|---|
| 1 Heizmodul mit Heizkörper | 1 Separations- und Heizmodul mit Heizkörper | |
| 2 Platine | | |
| 3 Akku/Batterie | | |
| 4 Vibrationsplatte | | |
| 5 Magnetstreifen | 5 einschaltbarer Elektromagnet | 5 einsetzbarer Magnet |
| 6 Separationsmodul | | |
| 7 Vertiefung für Reaktionsgefäße | | |

## Patentansprüche

1. Handgerät zur mobilen Isolierung von Nukleinsäuren, umfassend:
• mindestens einen Probenblock (1, 2) zum Einsetzen von Probengefäßen
• eine Probenblockaufnahme (6) mit Bohrungen oder Aussparungen für die Aufnahme der Probenblöcke (1, 2)
• eine Gerätebasis (7) mit elektronischen Steuereinheiten für die Probenblöcke, einer Spannungsquelle sowie einer Verbindung zur Probenblockaufnahme
• ein Testkit zur Isolierung von Nukleinsäuren,
**dadurch gekennzeichnet, dass**
a) die Probenblöcke (1, 2) mit der Probenblockaufnahme (6) und die Probenblockaufnahme (6) mit der Gerätebasis (7) über Steckverbinder oder einer Magnetverbindung verbunden sind und
b) sowohl die Probenblöcke (1, 2) von der der Probenblockaufnahme (6) als auch die Probenblockaufnahme (6) von der Gerätebasis (7) abnehmbar sind
c) und dass ein Probenblock ein Magnetseparationsmodul (2) umfasst.

2. Handgerät nach Anspruch 1, **dadurch gekennzeichnet, dass** die Probenblöcke (1, 2) mindestens ein Heizmodul (3) enthalten.

3. Handgerät nach Anspruch 1, **dadurch gekennzeichnet, dass** das Magnetseparationsmodul (2) ein oder mehrere Magneten in aufweist.

4. Handgerät nach Anspruch 1, **dadurch gekennzeichnet, dass** sich ein Sichtfenster im Probenblock (2) befindet.

5. Handgerät nach Anspruch 1, **dadurch gekennzeichnet, dass** die Probenblockaufnahme (6) eine Schüttel- oder Vibrationseinrichtung (5) sowie eine oder mehrere reversiblen Verbindungsstellen für die Wechselblöcke enthält.

6. Handgerät nach Anspruch 1, **dadurch gekennzeichnet, dass** die Probenblockaufnahme (6) eine zusätzliche Heizeinrichtung enthält.

7. Handgerät nach Anspruch 1, **dadurch gekennzeichnet, dass** als Spannungsquelle eine Batterie oder ein Akkumulator dient.

8. Handgerät nach einem der Ansprüche 1 bis 7, **dadurch gekennzeichnet, dass** es zusätzlich einen Anschluss für eine externe Spannungsquelle umfasst.

9. Handgerät nach einem der Ansprüche 1 bis 8, **dadurch gekennzeichnet, dass** es folgende Abmessungen besitzt:
• Höhe zwischen 6 und 10 cm, vorzugsweise 8 cm
• Tiefe zwischen 2 und 4 cm, vorzugsweise 3 cm
• Breite zwischen 4 und 8 cm, vorzugsweise 5 cm.

10. Handgerät nach einem der Ansprüche 1 bis 9, **dadurch gekennzeichnet, dass** das Heizmodul (1) gleichzeitig als Magnetseparationsmodul (2) dient und
a) dass sich neben diesem Heiz-Magnetseparationsmodul eine Aussparung zur Aufnahme eines Magneten befindet oder
b) dieses Heiz-Magnetseparationsmodul einen einschaltbaren Elektromagneten enthält.

11. Handgerät nach Anspruch 1, **dadurch gekennzeichnet, dass** das Testbesteck umfasst:
a) Einwegpipetten (z.B. Pasteurpipetten)
b) Flasche für Reagenzienabfall
c) Suspension mit magnetischen oder paramagnetischen Partikeln
d) Reaktionsgefäße
e) Lysepuffer sowie optional proteolytische Enzyme in flüssiger Form oder Reaktionsgefäße für die Lyse der Probe mit lagerstabilen festen Formulierungen an Lysereagenzien und proteolytischen Enzymen
f) Bindungspuffer
g) Waschpuffer
h) Elutionspuffer.

12. Transportbehälter, der das Handgerät gemäß einem der Ansprüche 1 bis 11 enthält.

13. Verwendung des Handgeräts gemäß einem der Ansprüche 1 bis 11 zur mobilen Isolierung von Nukleinsäuren, umfassend folgende Schritte:
• Lyse der Probe:
• Anbindung der DNA an magnetische Partikel:
• Separation der Magnetpartikel mit der gebundenen Nukleinsäure:
• Waschen der Magnetpartikel mit der gebundenen Nukleinsäure:
• Trocknung der Magnetpartikel:
• Elution der Nukleinsäure von den Magnetpartikeln.

## Claims

1. A handheld device for mobile isolation of nucleic acids comprising:
• at least one sample block (1, 2) for placing sample containers
• a sample block reception (6) with bores or recesses for receiving the sample blocks (1, 2)
• an equipment basis (7) with electronic control units for the sample blocks, a voltage source as well as a connection with the sample block reception
• a test kit for isolation of nucleic acids,
**characterised in that**
a) the sample blocks (1, 2) are connected with the sample block reception (6) and the sample block reception (6) is connected with the equipment basis (7) via plug-in connectors or a magnetic connection, and
b) not only the sample blocks (1, 2) can be removed from the sample block reception (6) but also the sample block reception (6) can be removed from the equipment basis (7)
c) and that a sample block comprises a magnetic separation module (2).

2. The handheld device according to claim 1, **characterised in that** the sample blocks (1, 2) contain at least one heating module (3).

3. The handheld device according to claim 1, **characterised in that** the magnetic separation module (2) comprises one or several magnets.

4. The handheld device according to claim 1, **characterised in that** there is an inspection window in the sample block (2).

5. The handheld device according to claim 1, **characterised in that** the sample block reception (6) includes shaking or vibration means (5) as well as one or several reversible connection points for the replacement blocks.

6. The handheld device according to claim 1, **characterised in that** the sample block reception (6) includes an additional heating means.

7. The handheld device according to claim 1, **characterised in that** a battery or an accumulator serves as a voltage source.

8. The handheld device according to any one of claims 1 to 7, **characterised in that** it comprises in addition a connection for an external voltage source.

9. The handheld device according to any one of claims 1 to 8, **characterised in that** it comprises the following dimensions:
• height between 6 and 10 cm, preferably 8 cm
• depth between 2 and 4 cm, preferably 3 cm
• width between 4 and 8 cm, preferably 5 cm.

10. The handheld device according to any one of claims 1 to 9, **characterised in that** the heating module (1) serves at the same time as a magnetic separation module (2) and
a) that adjacent to said heating magnetic separation module there is a recess for reception of a magnet or
b) said heating magnetic separation module contains a solenoid that can be switched on.

11. The handheld device according to claim 1, **characterised in that** the test instruments comprise:
a) disposable pipettes (e.g. Pasteur pipettes)
b) bottle for reagent waste
c) suspension with magnetic or paramagnetic particles
d) reaction vessels
e) lysis buffer as well as optionally proteolytic enzymes in liquid form or reaction vessels for the lysis of the sample with shelf stable solid formulations on lysis reagents and proteolytic enzymes
f) bonding buffer
g) detergent buffer
h) elution buffer.

12. A transport container which contains the handheld device according to any one of claims 1 to 11.

13. A use of the handheld device according to any one of claims 1 to 11 for mobile isolation of nucleic acids, comprising the following steps:
• lysis of the sample
• bonding of the DNA to magnetic particles
• separation of the magnetic particles with the bonded nucleic acid
• washing of the magnetic particles with the bonded nucleic acid
• drying of the magnetic particles
• elution of the nucleic acid from the magnetic particles.

## Revendications

1. Appareil portatif pour l'isolement mobile d'acides nucléiques comportant:
• au moins un bloc d'échantillon (1, 2) pour le placement des récipients d'échantillon
• une réception de bloc d'échantillon (6) avec des alésages ou des encoches pour la réception des blocs d'échantillon (1, 2)
• une base de l'appareil (7) avec des unités de contrôle électronique pour les blocs d'échantillon (6), une source de tension ainsi qu'un raccord à la réception des blocs d'échantillon
• un test kit pour l'isolement des acides nucléiques,
**caractérisé en ce que**
a) les blocs d'échantillon (1,2) sont raccordés à la réception de bloc d'échantillon (6) et la réception de bloc d'échantillon (6) est raccordée à la base de l'appareil (7) par des connecteurs ou un raccord magnétique et
b) non seulement les blocs d'échantillon (1, 2) sont amovibles de la réception de bloc d'échantillon (6) mais aussi la réception de bloc d'échantillon (6) est amovible de la base de l'appareil (7)
c) un bloc d'échantillon comprend un module magnétique de séparation (2)

2. Appareil portatif selon la revendication 1, **caractérisé en ce que** les blocs d'échantillon (1,2) comprennent au moins un module de chauffage (3).

3. Appareil portatif selon la revendication 1, **caractérisé en ce que** le module magnétique de séparation (2) comprend un ou plusieurs aimants.

4. Appareil portatif selon la revendication 1, **caractérisé en ce qu'**il y a une fenêtre d'observation dans le bloc d'échantillon (2).

5. Appareil portatif selon la revendication 1, **caractérisé en ce que** la réception de bloc d'échantillon (6) comprend un agitateur ou un vibrateur (5) ainsi que une ou plusieurs jonctions réversibles pour les blocs interchangeables.

6. Appareil portatif selon la revendication 1, **caractérisé en ce que** la réception de bloc d'échantillon (6) comprend un dispositif de chauffage supplémentaire.

7. Appareil portatif selon la revendication 1, **caractérisé en ce qu'**une batterie ou un accumulateur sert de source de tension.

8. Appareil portatif selon l'une quelconque des revendications précédentes, **caractérisé en ce qu'**il comprend également un raccord pour une source de tension externe.

9. Appareil portatif selon l'une quelconque des revendications 1 à 8, **caractérisé en ce qu'**il comprend les dimensions suivantes:
- hauteur entre 6 et 10 cm, de préférence 8 cm
- profondeur entre 2 et 4 cm, de préférence 3 cm
- largeur entre 4 et 8 cm, de préférence 5 cm.

10. Appareil portatif selon l'une quelconque des revendications 1 à 9, **caractérisé en ce que** le module de chauffage (1) sert en même temps de module magnétique de séparation (2) et
a) que adjacent audit module magnétique de séparation il y a une encoche pour la réception d'un aimant ou
b) ledit module magnétique de séparation et de chauffage comprend un électroaimant qui peut être enclenché.

11. Appareil portatif selon la revendication 1, **caractérisé en ce que** le kit permettant d'effectuer les tests comprend:
a) des pipettes jetables (par exemple pipettes Pasteur)
b) une bouteille pour des déchets de réactifs
c) une suspension avec des particules magnétiques ou paramagnétiques
d) des récipients de réaction
e) un tampon de lyse ainsi que facultativement des enzymes protéolytiques en forme liquide ou des récipients de réaction pour la lyse de l'échantillon avec des formulations solides de longue conservation aux réactifs de lyse et des enzymes protéolytiques
f) un tampon de liaison
g) un tampon détergent
h) un tampon d'élution.

12. Conteneur de transport comportant l'appareil portatif selon l'une quelconque des revendications 1 à 11.

13. Utilisation de l'appareil portatif selon l'une quelconque des revendications 1 à 11 pour l'isolement mobile des acides nucléiques comportant les étapes suivantes:
- la lyse de l'échantillon
- la liaison de la ADN aux particules magnétiques
- la séparation des particules magnétiques avec l'acide nucléique lié
- le lavage des particules magnétiques avec l'acide nucléique lié
- le séchage des particules magnétique
- l'élution de l'acide nucléique des particules magnétiques.
